# EUROPÄISCHE PATENTANMELDUNG

(11) **EP 0 576 862 A2**
(43) Veröffentlichungstag der Anmeldung: **05.01.1994**
(21) Anmeldenummer: 93109016.1
(22) Anmeldetag: 04.06.1993
(51) Int. Cl.: C12N 15/11, C12N 5/10, C12Q 1/02

(54) **DNA zur Immortalisierung von humanen oder tierischen Zellen**

(30) Priorität: 10.06.1992 DE 4218945
(71) Anmelder: BOEHRINGER MANNHEIM GMBH, D-68298 Mannheim (DE)
(72) Erfinder: Abken, Hinrich Johann, Dr., D-53332 Bornheim (DE); Willecke, Klaus, Prof. Dr., D-53639 Königswinter (DE); Jungfer, Herbert, Dr., W-8130 Starnberg (DE); Barchet, Heinrich, W-8139 Bernried (DE)

(57) **Zusammenfassung**

Die Erfindung betrifft DNA-Sequenzen, welche zur Immortalisierung von humanen oder tierischen Zellen geeignet sind, Verfahren zur Gewinnung von solchen DNA-Sequenzen, sowie Verfahren zur Herstellung von immortalisierten humanen oder tierischen Zellen unter Verwendung einer solchen DNA.

## Beschreibung

Die Erfindung betrifft DNA-Sequenzen, welche zur Immortalisierung von humanen oder tierischen Zellen geeignet sind, Verfahren zur Gewinnung von solchen DNA-Sequenzen, sowie Verfahren zur Herstellung von immortalisierten humanen oder tierischen Zellen unter Verwendung einer solchen DNA.

Normale menschliche und tierische Zellen haben in Kultur nur eine begrenzte Lebens- und Teilungsfähigkeit. Der Beginn der Zellalterung in der Kultur ist vom Differenzierungsgrad der Zelle, vom Alter und der Spezies des Spenders, sowie von der Kulturdauer abhängig. Alternde Zellen stellen die Zellteilung und die Synthese von speziellen Zellprodukten wie z.B. Antikörpern, Hormonen oder Wachstumsfaktoren ein. Die aus solchen normalen menschlichen und tierischen Zellen gewonnenen Zellprodukte haben gegenüber rekombinant hergestellten Produkten immer dann erhebliche Vorteile, wenn z.B. differenzierungs- und/oder speziesspezifische sekundäre Modifikationen wie z.B. eine Glycosylierung, für die biologische Wirksamkeit von Bedeutung sind. Sogenannte Zellinien vermehren sich unbegrenzt in der Kultur. Dies erlaubt eine Züchtung dieser Zellinien in beliebiger Anzahl und Gewinnung von Zellprodukten in unbegrenzter Menge. Da die meisten Zellinien aus bösartigen Tumoren etabliert wurden, prägen diese permanent teilungsfähigen Zellen jedoch zahlreiche Eigenschaften von Tumorzellen aus und haben andererseits einige Eigenschaften von normalen, nur begrenzt züchtbaren Zellen, wie insbesondere auch die Fähigkeit zur Produktion von speziellen Zellprodukten, verloren. Zur Gewinnung von Zellprodukten aus hochdifferenzierten Zellen sind daher auch Verfahren entwickelt worden, mit denen normale, nicht tumorigene Zellen als Zellinien durch Immortalisierung über lange Zeit in Kultur gehalten werden können.

Langzeitkulturen von menschlichen B-Lymphozyten sowie T-Lymphozyten können durch wiederholte Stimulierung durch zur Zellteilung anregende Substanzen (Mitogene) aufrechterhalten werden. Als Mitogene werden hierbei z.B. polyklonale B-Zell Aktivatoren, Phorbolester zusammen mit einem Kalziumionophor oder spezifische Wachstumsfaktoren (Cytokine) in Kombination mit immobilisierten Anti-B-Zell-Antikörpern verwendet (J. Immunol. 141 (1988), 164; Science 251 (1991), 70 und J. Exp. Med. 170 (1989), 877). Bei diesen Verfahren bleiben die Zellen jedoch in ihrem Wachstum von der Zugabe von Cytokinen oder von wiederholten mitogenen Stimulierungen abhängig.

Ein anderes Verfahren zur Immortalisierung humaner oder tierischer Zellen besteht in der Infektion derartiger Zellen mit transformierenden Viren. Dabei verlieren die Zellen aber einige Eigenschaften normaler Zellen und prägen zusätzlich Eigenschaften von permanent teilungsfähigen Tumorzellen aus. So verharren z.B. Lymphozyten nach einer Infektion mit dem Epstein-Barr-Virus (EBV) in einer frühen Differenzierungsstufe der B-Zell-Entwicklung. Nach EBV Infektion menschlicher B-Lymphozyten in vitro werden deshalb häufig Zellklone erhalten, die IgM in sehr geringen Mengen oder gar keine Immunglobuline produzieren. Bei weiterer Kultur in vitro verlieren auch die zunächst Immunglobuline produzierenden EBV-infizierten B-Zellen häufig die Fähigkeit, Immunglobulin zu synthetisieren. Aus diesem Grund sind EBV-infizierte B-Lymphozyten wenig geeignet, spezifische Antikörper in größeren Mengen bei gewünschter konstanter Syntheseleistung der Zellen über eine lange Kulturdauer zu gewinnen.

Das gleiche gilt für die Immortalisierung von Maus-B-Lymphozyten durch Infektion mit dem Abelson Leukämie Virus.

Eine weitere Technik zur Immortalisierung von Zellen ist die Zell-Zell-Fusion (Köhler und Milstein, Nature 256 (1975), 495). Hierbei werden z.B. normale Lymphozyten mit begrenzter Teilungsfähigkeit mit permanent wachsenden Zellinien (Myelomzellinien, Lymphomzellinien, Epstein-Barr-Virus infizierte lymphoblastoide Zellinen) fusioniert. Durch geeignete Selektionsmedien wird gewährleistet, daß die erhaltenen Hybridzellen sich bevorzugt vermehren können, die Fusionspartner selbst aber absterben. Durch Anlegen von Einzelzellkulturen können die gewonnenen Hybridzellen kloniert und die Zellklone, die das gewünschte Produkt sezernieren, für die Massenproduktion vermehrt werden. Da die Hybridzellen den doppelten Chromosomensatz der Ausgangszelle haben, neigen sie dazu, in den folgenden Zellgenerationen Chromosomen (meist die der normalen Ausgangszellen) zu verlieren. Dabei können die gewünschten Eigenschaften der Hybridzellen (insbesondere Produktion des gewünschten Zellprodukts) wieder verloren gehen. Nachteile der Hybridomtechnik sind somit die aufwendige Selektion der gewünschten Hybridzellen, Synthese von hybriden Zellprodukten, wenn es sich hierbei um mehrkettige Proteine handelt, sowie Chromosomenverlust, der zu einem Verlust der Synthese des gewünschten Zellprodukts führen kann.

Eine Verbesserung dieses Verfahrens wird in der EP-B 0 093 436 beschrieben. Danach werden Zellen durch Fusion mit einem nicht vermehrungsfähigen Fragment einer transformierten Zelle immortalisiert. Die entstehende Fusionszelle ist in Kultur unbegrenzt teilungsfähig. Als nicht vermehrungsfähiges Fragment dienen hierbei Fraktionen des Zytoplasmas, bevorzugt Zytoplasten. Da die verwendeten Zytoplasmafragmente sich nicht in Kultur vermehren, erübrigt sich eine Selektion der Hybridzelle.

Weiterhin treten die oben erwähnten Nachteile der Zell-Zell-Hybride, wie Chromosomenverlust und damit Verlust von gewünschten Zelleigenschaften, sowie die Synthese von hybriden Zellprodukten bei den so erhaltenen Zellinien nicht auf. In der EP-A 0 256 512 wird eine Weiterentwicklung dieses Verfahrens beschrieben. Hierbei wird zunächst eine DNA-Fraktion aus dem Zytoplasma oder bevorzugt aus den Zytoplasten permanent teilungsfähiger Zellen isoliert. Mit dieser DNA werden dann die zu immortalisierenden Zellen transfiziert. Hierdurch wird eine Steigerung der Ausbeute immortalisierter Zellen erreicht. Auch dieses Verfahren führt zur Etablierung von immortalisierten Zellinien unter Vermeidung der Nachteile der Hybridomatechnik. Jedoch ist die Isolierung der immortalisierenden DNA aus einer Mitochondrienfreien Zytoplasmafraktion aufwendig und erfordert zahlreiche Reinigungsschritte. Weiterhin ist die Ausbeute der so gewonnenen DNA gering. So können z.B. mit Hilfe geeigneter Anreicherungsverfahren maximal 200 ng DNA aus dem Zytoplasma von 10⁹ L929-Zellen isoliert werden. Diese Menge DNA reicht aber nur für die Etablierung von ca. 30 Zellklonen nach Transfektion von 10⁸ Lymphozyten.

Aufgabe der Erfindung ist es daher, eine DNA zur Immortalisierung von humanen oder tierischen Zellen in großen Mengen und in reiner Form zur Verfügung zu stellen.

Diese Aufgabe wird gelöst durch eine DNA, welche zur Immortalisierung von humanen oder tierischen Zellen geeignet ist und dadurch erhältlich ist, daß man eine Mitochondrien-freie Zytoplasmafraktion aus permanent züchtbaren humanen oder tierischen Zellen gewinnt, diese Zytoplasmafraktion mit RNase und Proteinase behandelt, daraus eine DNA-Fraktion isoliert, welche in einem CsCl-Gradienten eine Dichte von etwa 1,86 g/cm³ aufweist und aus dieser DNA-Fraktion eine DNA isoliert, welche mit der in SEQ ID NO 1 und/oder SEQ ID NO 2 gezeigten DNA-Sequenz hybridisiert.

Ein bevorzugter Gegenstand der Erfindung ist eine DNA, welche die in SEQ ID NO 1 angegebene Sequenz aufweist, sowie eine DNA, welche die in SEQ ID NO 2 angegebene Sequenz aufweist.

Es hat sich überraschenderweise gezeigt, daß mit diesen DNA-Sequenzen eine effiziente Immortalisierung von humanen oder tierischen Zellen möglich ist. Durch die Klonierung der erfindungsgemäßen, immortalisierenden DNA ist diese DNA in beliebiger Menge für die Immortalisierung von humanen oder tierischen Zellen verfügbar.

Diese DNA-Sequenzen können aus dem Zytoplasma von permanent züchtbaren humanen oder tierischen Zellen gewonnen werden. Vorzugsweise werden hierfür Zytoplasten von Tumorzellen verwendet. Besonders bevorzugt sind Zytoplasten transformierter Maus-L-Zellen (L929-Zellen, ATCC CCL 1) oder Ehrlich Aszites Zellen (EAZ, ATCC CCL 77). Es können jedoch auch andere, dem Fachmann geläufige Tumorzellinien, sowie immortalisierte, nicht tumorigene Zellinien verwendet werden. Die Zytoplasten werden nach bekannten Verfahren (Wigler und Weinstein, Biochem. Biophys. Res. Comm. 63 (1975), 669-674) gewonnen und lysiert. Die Lyse erfolgt z.B. durch wiederholtes Einfrieren und Auftauen oder aber durch Lyse der Zellen in Gegenwart von 50 mmol/l Tris-HCl pH 7,5, 10 mmol/l EDTA, 1,5 mmol/l MgCl₂. Die Mitochondrien werden aus der Zytoplastenfraktion abgetrennt, vorzugsweise mit Hilfe eines Sucrosegradienten (J. Biol. Chem. 249 (1974), 7991-7995). Die Fraktionen, die keine Mitochondrien enthalten, werden mit RNase, vorzugsweise mit RNaseA und RNaseT1, sowie anschließend mit Proteinase, vorzugsweise mit Proteinase K inkubiert. Aus diesem Ansatz wird die DNA nach bekannten Methoden isoliert und eine Fraktion, welche in einem Cäsiumchloridgradienten eine Dichte von 1,86 g/cm³ aufweist, angereichert. Bevorzugt erfolgt diese Anreicherung mit Hilfe eines CsCl-Dichtegradienten oder über eine elektrophoretische Auftrennung nach dem Fachmann geläufigen Verfahren (Sambrook et al. Molecular Cloning, Cold Spring Harbor Laboratory, Second Edition 1989). Die hierbei gewonnene DNA-Fraktion wird in dem Fachmann geläufigen Vektoren, wie z.B. pUC19, kloniert und in dem Fachmann ebenfalls geläufigen Wirtsorganismen, wie z.B. E.coli HB101 oder DH5α vermehrt. Die rekombinanten Plasmide, welche eine erfindungsgemäße DNA-Sequenz enthalten, werden durch Hybridisierung mit der in SEQ ID NO 1 oder der in SEQ ID NO 2 gezeigten DNA-Sequenz identifiziert. Es hat sich dabei überraschenderweise gezeigt, daß nur einer von 28 unabhängigen rekombinanten Plasmidklonen der DNA-Fraktion einer Dichte von 1,86 g/cm³ aus L929-Zellen (pLC108), sowie einer aus 25 rekombinanten Plasmidklonen der entsprechenden DNA-Fraktion aus Ehrlich Aszites Zellen (pEFC38) eine DNA enthält, welche zur Immortalisierung von humanen oder tierischen Zellen geeignet ist. Die Sequenz dieser DNA ist in SEQ ID NO 1 (Insert von pLC108), bzw. in SEQ ID NO 2 (Insert von pEFC38) gezeigt. Mit Hilfe dieser Sequenzen ist es nun leicht möglich, weitere zur Immortalisierung von humanen oder tierischen Zellen geeignete DNA-Sequenzen aufzufinden.

Ein weiterer Gegenstand der Erfindung ist daher ein Verfahren zur Gewinnung einer DNA, welche zur Immortalisierung von humanen oder tierischen Zellen geeignet ist, wobei dieses Verfahren dadurch gekennzeichnet ist, daß man eine Mitochondrien-freie Zytoplasmafraktion aus permanent züchtbaren humanen oder tierischen Zellen gewinnt, diese Zytoplasmafraktion mit RNase und Proteinase behandelt, daraus eine Fraktion isoliert, welche in einem CsCl-Gradienten eine Dichte von etwa 1,86 g/cm³ aufweist und aus dieser DNA-Fraktion eine DNA isoliert, welche mit der in SEQ ID NO 1 und/oder SEQ ID NO 2 gezeigten DNA-Sequenz hybridisiert.

Die zur Gewinnung dieser DNA verwendete Zytoplasmafraktion kann wie oben beschrieben, durch wiederholtes Einfrieren und Auftauen oder durch Lyse der Zellen in Gegenwart von 50 mmol/l Tris-HCl pH 7,5, 10 mmol/l EDTA, 1,5 mmol/l MgCl₂ gewonnen werden. Alternativ kann auch eine Zellfraktion verwendet werden, die frei von nukleärer DNA ist. Eine solche Fraktion gewinnt man bevorzugt durch Lyse der Zellen mit NaCl und SDS (Hirt Extraktion, J. Mol. Biol. 26 (1967), 365-369) und anschließender Zentrifugation. Aus dem Überstand können dann die immortalisierenden DNA-Sequenzen gewonnen werden.

Weiterhin können die erfindungsgemäßen DNA-Sequenzen auch aus einer genomischen Genbank oder einer cDNA-Bank von menschlichen oder tierischen Zellen gewonnen werden. Hierbei werden die in SEQ ID NO 1 und/oder SEQ ID NO 2 gezeigten DNA-Sequenzen als Hybridisierungssonde zur Identifizierung einer erfindungsgemäßen DNA-Sequenz in der Genbank verwendet. Dabei können sowohl Genbanken aus permanent wachsenden Zellen, als auch solche aus normalen nicht immortalisierten Zellen verwendet werden.

Ein weiterer Gegenstand der Erfindung ist daher ein Verfahren zur Gewinnung einer DNA, welche zur Immortalisierung von tierischen oder humanen Zellen geeignet ist, wobei dieses Verfahren dadurch gekennzeichnet ist, daß man eine genomische Gen-Bank oder eine cDNA-Bank von menschlichen oder tierischen Zellen mit der in SEQ ID NO 1 oder SEQ ID NO 2 gezeigten DNA-Sequenz hybridisiert und die mit einer dieser DNA-Sequenzen hybridisierende DNA isoliert.

Ein weiterer Gegenstand der Erfindung ist ein Verfahren zur Gewinnung der erfindungsgemäßen DNA-Sequenzen, welches dadurch gekennzeichnet ist, daß man eine DNA- oder RNA-Fraktion von menschlichen oder tierischen Zellen enzymatisch vermehrt, wobei Oligonukleotid-Starter Moleküle verwendet werden, welche zu der in SEQ ID NO 1 und/oder SEQ ID NO 2 gezeigten Sequenz homolog sind. Vorzugsweise werden Oligonukleotid-Starter Moleküle verwendet, welche eine Länge von 20 bis 30 Basenpaaren aufweisen. Die enzymatische Vermehrung der immortalisierenden DNA-Sequenzen mit Hilfe der Polymerase-Ketten-Reaktion ("DNA polymerase chain reaction", PCR) erfolgt gemäß dem Fachmann bekannten Verfahren (Mullis und Fallona, Meth. Enzymol. 155 (1987), 335 - 350; Saiki et al., Science 239 (1988), 487 - 491).

Dabei können als DNA-Fraktion sowohl Genbanken aus permanent wachsenden Zellen, als auch solche aus normalen nicht immortalisierten Zellen verwendet werden. Weiterhin ist bei diesem Verfahren auch die Verwendung einer DNA- oder RNA-Präparation aus beliebigen humanen oder tierischen Zellen möglich. Die Sequenzen von besonders bevorzugten Oligonukleotid-Starter Molekülen für die Vermehrung der in SEQ ID NO 1 gezeigten DNA-Sequenz sind in SEQ ID NO 3 und 4 gezeigt. Die Sequenzen von besonders bevorzugten Oligonukleotid-Starter Molekülen für die Vermehrung der in SEQ ID NO 2 gezeigten DNA-Sequenz sind in SEQ ID NO 5 und 6 angegeben.

Schließlich können die erfindungsgemäßen DNA-Sequenzen auch mit handelsüblichen Oligonucleotidsynthese-Apparaturen chemisch synthetisiert werden.

Ein weiterer Gegenstand der Erfindung ist daher ein Verfahren zur Gewinnung einer DNA, welche zur Immortalisierung von humanen oder tierischen Zellen geeignet ist, wobei dieses Verfahren dadurch gekennzeichnet ist, daß man die in SEQ ID NO 1 oder SEQ ID NO 2 gezeigte DNA-Sequenz chemisch synthetisiert.

Ein weiterer Gegenstand der Erfindung ist ein Verfahren zur Herstellung von immortalisierten humanen oder tierischen Zellen durch Transfektion von normalen humanen oder tierischen Zellen mit einer erfindungsgemäßen DNA-Sequenz oder einem Teil dieser Sequenz und Züchtung der transfizierten Zellen in einem Kulturmedium ohne Selektionssubstanzen. Es hat sich hierbei überraschenderweise gezeigt, daß bereits 10 - 30 ng einer erfindungsgemäßen DNA ausreichen, um bei Transfektion von 10⁸ Lymphozyten 30 - 40 immortalisierte lymphoide Kolonien zu erhalten. Eine weitere Verbesserung der immortalisierenden Wirkung um den Faktor 3-6 konnte überraschenderweise erreicht werden, wenn die erfindungsgemäßen DNA-Sequenzen vor der Transfektion mit einer Proteinfraktion von permanent züchtbaren Zellen inkubiert wurden.

Ein weiterer Gegenstand der Erfindung ist daher ein Verfahren zur Herstellung von immortalisieten humanen oder tierischen Zellen durch Transfektion von normalen humanen oder tierischen Zellen mit einer erfindungsgemäßen DNA-Sequenz oder einem Teil dieser Sequenz, welche zuvor mit einer Proteinfraktion aus permanent züchtbaren humanen oder tierischen Zellen inkubiert wurde, wobei als Proteinfraktion ein 25000 x g Überstand der Kernfraktion oder ein 100000 x g Überstand der Zytoplasmafraktion verwendet wird, der mit RNase vorbehandelt wurde.

Die hierzu verwendete Proteinfraktion wird aus dem Kern bzw. aus dem Zytoplasma der permanent züchtbaren Zellen gewonnen. Hierzu werden zunächst die Zellen durch wiederholtes Einfrieren und Auftauen lysiert. Die Auftrennung in Kern- und Zytoplasmafraktion erfolgt durch 30-minütige Zentrifugation bei 6000 x g. Die sedimentierten Zellkerne werden in Puffer aufgenommen und durch Ultraschallbehandlung homogenisiert. Nach 30-minütiger Zentrifugation bei 25000 x g und 4°C, Dialyse des Überstands gegen PBS und anschließendem RNase Verdau, wird der für das erfindungsgemäße Verfahren verwendete 25000 x g Überstand der Kernfraktion erhalten. Die Zytoplasmafraktion wird eine Stunde bei 100000 x g und 4°C zentrifugiert, der Überstand ebenfalls gegen PBS dialysiert und mit RNase verdaut.

Die Vorinkubation der erfindungsgemäßen DNA mit dem 25000 x g Überstand der Kernfraktion bzw. dem 100000 x g Überstand der Zytoplasmafraktion erfolgt für 30 Minuten bei 4°C in 10 mmol/l Tris-HCl pH 7,0, 10 mmol/l β-Mercaptoethanol, 10 ng/ml tRNA, 66 ng/ml Heringssperma DNA (1 mM ATP, 1 mM GTP), sowie entweder 80 mmol/l NaCl (sofern eine DNA verwendet wird, welche mit der in SEQ ID NO 2 gezeigten DNA-Sequenz hybridisiert) oder mit 8 mmol/l ZnCl₂ (sofern eine DNA verwendet wird, welche mit der in SEQ ID NO 1 gezeigten DNA-Sequenz hybridisiert). Anschließend werden diejenigen Proteine, welche nicht an die jeweilige DNA-Sequenz gebunden haben, durch einstündige Inkubation mit Proteinase K (20 µg/ml) bei 60°C abgebaut.

Die Bindung der zurückbehaltenen Proteinfraktion an die erfindungsgemäßen DNA-Sequenzen ist stabil gegen hohe Salzkonzentrationen wie 6,9 mol/l CsCl und gegen weiteren Proteinase K-Verdau (100 µg/ml, 1 Stunde bei 60°C). Die erhaltenen DNA-Protein Partikel sind somit stabiler als die von den Chromosomen des Zellkerns bekannten Nukleosomen. Dennoch ist eine Amplifikation der in diesen Partikeln gebundenen DNA mit Hilfe der Taq-Polymerase möglich.

Die erfindungsgemäßen DNA-Sequenzen können in den dem Fachmann geläufigen Klonierungssystemen kloniert und vermehrt werden. Sie sind auch nach der Klonierung noch in der Lage, humane oder tierische Zellen zu immortalisieren.

Ein weiterer Gegenstand der Erfindung ist daher ein Verfahren zur Herstellung von immortalisierten humanen oder tierischen Zellen durch Transfektion von normalen humanen oder tierischen Zellen mit einer klonierten erfindungsgemäßen DNA-Sequenz oder einem Teil dieser Sequenz. Es können hierbei auch solche erfindungsgemäßen DNA-Sequenzen verwendet werden, bei denen einzelne Basen der genannten DNA-Sequenzen duch andere oder durch modifizierte Basen ersetzt sind. Hierdurch kann z.B. eine höhere Stabilität der erfindungsgemäßen DNA-Sequenzen erreicht werden.

Es hat sich überraschenderweise gezeigt, daß die DNA einer nach einem der oben genannten Verfahren immortalisierten Zelle ebenfalls zur Immortalisierung von humanen oder tierischen Zellen geeignet ist.

Ein weiterer Gegenstand der Erfindung ist daher ein Verfahren zur Herstellung von immortalisierten humanen oder tierischen Zellen durch Transfektion von normalen humanen oder tierischen Zellen mit der Gesamt-DNA aus einer nach einem der erfindungsgemäßen Verfahren erhältlichen immortalisierten Zelle.

Es hat sich weiterhin überraschenderweise gezeigt, daß eine bessere Ausbeute an immortalisierten lymphoiden Kolonien erhalten wird, wenn sich die zu immortalisierende Zelle in einem zum Wachstum angeregten Zustand befindet.

Ein weiterer Gegenstand der Erfindung ist daher eine besondere Ausführungsform des erfindungsgemäßen Verfahrens zur Herstellung von immortalisierten humanen oder tierischen Zellen, wobei man die zu immortalisierende Zelle vor der Transfektion mit der erfindungsgemäßen DNA in einen zur Teilung angeregten Zustand versetzt. Die Überführung von ruhenden Zellen in einen solchen zur Teilung angeregten Zustand (Proliferationsphase I, J. Cell Biol. 103 (1986), 795-805) erfolgt nach dem Fachmann geläufigen Methoden, z.B. durch Zugabe von "Pokeweed Mitogen" oder anderen Mitogenen oder Antigenen zu dem Kulturmedium.

Die mit der erfindungsgemäßen DNA oder nach dem erfindungsgemäßen Verfahren immortalisierten menschlichen Lymphozyten prägen keinen neoplastisch transformierten Phänotyp aus. So bilden diese Zellen keine Kolonien in halbfestem Agar-Medium. Tumorzellen wachsen dagegen in einem solchen Medium.

Durch Inkubation der immortalisierten Zellen mit karzinogenen Substanzen in vitro oder durch Bestrahlung mit Teilchen-Strahlen oder elektromagnetischen Strahlen kann ein Wachstum in halbfestem Agar-Medium induziert werden. Die Koloniebildung der immortalisierten Zellen in diesem Medium ist somit ein Hinweis auf eine mögliche transformierende Aktivität der entsprechenden Substanz bzw. der verwendeten Teilchen-Strahlen oder elektromagnetischen Strahlen.

Ein weiterer Gegenstand der Erfindung ist schließlich die Verwendung einer nach dem erfindungsgemäßen Verfahren erhaltenen immortalisierten Zelle zur Gewinnung von homologen oder heterologen Zellprodukten, zur Prüfung von Wirksubstanzen oder von Teilchen-Strahlen oder elektromagnetischen Strahlen.

Die Erfindung wird durch die folgenden Beispiele in Verbindung mit den Sequenzprotokollen näher erläutert.
- SEQ ID NO 1:: Zeigt eine für die Immortalisierung von humanen oder tierischen Zellen geeignete DNA-Sequenz aus L929-Zellen.
- SEQ ID NO 2:: Zeigt eine für die Immortalisierung von humanen oder tierischen Zellen geeignete DNA-Sequenz aus Ehrlich-Ascites-Zellen.
- SEQ ID NO 3:: Zeigt einen zur Amplifikation einer DNA der in SEQ ID NO 1 gezeigten Sequenz geeigneten Primer.
- SEQ ID NO 4:: Zeigt einen zur Amplifikation der DNA der in SEQ ID NO 1 gezeigten Sequenz geeigneten Primer.
- SEQ ID NO 5:: Zeigt einen zur Amplifikation einer DNA der in SEQ ID NO 2 gezeigten Sequenz geeigneten Primer.
- SEQ ID NO 6:: Zeigt einen zur Amplifikation einer DNA der in SEQ ID NO 2 gezeigten Sequenz geeigneten Primer.

### Beispiel 1

Isolierung einer DNA, welche zur Immortalisierung von normalen humanen oder tierischen Zellen geeignet ist.

Zur Isolierung einer DNA, welche zur Immortalisierung von normalen humanen oder tierischen Zellen werden zunächst Zytoplasten transformierter Maus-L-Zellen (L929-Zellen, ATCC CCL 1) bzw. von Ehrlich Aszites Zellen (EAZ, ATCC CCL 77) nach bekannten Verfahren (Wigler und Weinstein, Biochem. Biophys. Res. Comm. 63 (1975), 669-674) gewonnen und lysiert. Die Lyse erfolgt durch wiederholtes Einfrieren und Auftauen. Die Mitochondrien werden aus der Zytoplastenfraktion mit Hilfe eines Sucrosegradienten (J. Biol. Chem. 249 (1974), 7991-7995) abgetrennt. Die Fraktionen, die keine Mitochondrien enthalten, werden mit RNaseA (20µg/ml) und RNaseT1 (1000 U/ml), sowie anschließend mit Proteinase K (50 µg/ml) inkubiert. Aus diesem Ansatz wird die DNA nach bekannten Methoden isoliert und eine Fraktion, welche in einem Cäsiumchloridgradienten eine Dichte von 1,86 g/cm³ aufweist, über einen CsCl-Dichtegradienten (Sambrook et al. Molecular Cloning, Cold Spring Harbor Laboratory, Second Edition 1989) angereichert. Die hierbei gewonnene DNA-Fraktion wird in pUC19 kloniert und in E.coli HB101 vermehrt.

Einer von 28 erhaltenen unabhängigen rekombinanten Plasmidklonen der DNA-Fraktion einer Dichte von 1,86 g/cm³ aus L929-Zellen (pLC108), sowie einer aus 25 erhaltenen rekombinanten Plasmidklonen der entsprechenden DNA-Fraktion aus Ehrlich Aszites Zellen (pEFC38) enthält eine DNA, welche zur Immortalisierung von humanen oder tierischen Zellen geeignet ist. Die Sequenz dieser DNA ist in SEQ ID NO 1 (Insert von pLC108), bzw. in SEQ ID NO 2 (Insert von pEFC38) gezeigt.

### Beispiel 2

Immortalisierung menschlicher Lymphozyten aus dem peripheren Blut durch Transfektion mit LC108-DNA (SEQ ID NO 1) bzw. EFC38-DNA (SEQ ID NO 2)

### 1. Isolierung menschlicher Lymphozyten aus dem peripheren Blut.

Peripheres Blut erwachsener Spender wird mit dem dreifachen Volumen eines Puffers aus 3 mmol/l Zitronensäure, 100 mmol/l Dextrose, 70 mmol/l NaCl und 30 mmol/l Na-Citrat, pH 6.1 verdünnt. Die Lymphozyten werden von Erythrozyten und von anderen kernhaltigen Zellen durch Zentrifugation in einem LymphoprepR-Gradienten (Metrizoat, Nyegaard, Oslo) bei 400 g für 35 min abgetrennt (A. Boyum, Scand. J. Clin. Invest. 21, Suppl. 97 (1968), 51 - 76). Die erhaltenen Lymphozyten werden zweimal in "phosphate buffered saline" (PBS, 136 mmol/l NaCl, 2,7 mmol/l KCl, 1,5 mmol/l KH₂PO₄, 6,5 mmol/l Na₂HPO₄, 0,4 mmol/l MgSO₄, 0,7 mmol/l CaCl₂, pH 7,4) gewaschen und in Iscove's DMEM mit 4 mmol/l Glutamin, 1 mmol/l Pyruvat, 1 mmol/l Oxalessigsäure, 0,1 U/ml Insulin, 10 µg/ml Transferrin und 10% fötalem Kälberserum bei einer Zelldichte von 3 x 10⁶ Zellen/ml bei 37°C, 5 % CO₂ kultiviert.

### 2. Übertragung der immortalisierenden DNA-Sequenzen in menschliche Lymphozyten.

Die gemäß Beispiel 2.1 präparierten Lymphozyten (3 x 10⁸ Zellen) werden durch "pokeweed mitogen" (PWM, 5µg/ml) zur Zellteilung angeregt und einen Tag später in drei Parallelkulturen (A, B, C) zu je 10⁸ Zellen aufgeteilt. Die Zellen der Kultur A werden mit pLC108 DNA, die Zellen der Kultur B mit pEFC38 DNA inkubiert. Die hier verwendeten Plasmide pLC108 und pEFC38 enthalten die in SEQ ID NO 1 bzw. SEQ ID NO 2 gezeigte DNA-Sequenz, kloniert in der BamHI site von pUC 19. Die Zellen der Kultur C werden genauso behandelt wie die Kulturen A und B, jedoch ohne daß eine zu übertragende DNA im Inkubationsmedium vorhanden ist.

Auf einer Polystyrol-Petrischale werden 30 µl Transfektionsreagenz DOTAP (1 mg/ml, Boehringer Mannheim GmbH, Kat. Nr. 1202 375) mit 70 µl HBS (20 mmol/l Hepes, 150 mmol/l NaCl, pH 7,4) versetzt. Die DNA (200 ng) wird in 100 µl HBS gelöst, zu dem verdünnten Transfektionsreagenz gegeben und 10 min bei Raumtemperatur inkubiert. Die zu transfizierenden Lymphozyten (10⁸ Zellen in 3 ml Iscove's DMEM) werden hinzugegeben. Dieser Ansatz wird über Nacht bei 37°C, 5% CO₂ inkubiert. Die Zellen werden einmal mit PBS gewaschen, in einer Dichte von 10⁷ Zellen/ml in Iscove's DMEM mit 4 mmol/l Glutamin, 1 mmol/l Pyruvat, 1 mmol/l Oxalessigsäure, 0,1 U/ml Insulin, 10 µg/ml Transferrin und 10 % fötalem Kälberserum in 96-er Mikrotiterplatten (100 µl/Vertiefung) ausgesät und bei 37°C, 5% CO₂ kultiviert. Während der nächsten zwei Wochen werden 50 µl Kulturmedium durch frisches Medium ersetzt. In der 4. Woche wird 100 µl frisches Medium hinzugegeben. In der 5. Woche zeigen sich permanent wachsende Kolonien, die in der 6. Woche in eine 24-er Petrischale überführt werden. Die jetzt auswachsenden immortalisierten Zell-Linien können routinemäßig alle 7 Tage in Iscove's DMEM subkultiviert werden.

In den Kulturen A und B werden 5 Tage nach DNA-Übertragung Lymphozyten-Kolonien beobachtet, die in den folgenden Tagen an Größe und Zell-Zahl (bis zu 1000 Zellen/Kolonie) zunehmen. Diese Kolonien zerfallen dann in mehrere kleinere Kolonien, wovon einige etwa ab der 3. bis 4. Woche nach DNA-Übertragung kontinuierlich zu wachsen beginnen. In der 5. Woche nach DNA-Übertragung sind in allen Vertiefungen der Mikrotiterplatte Kolonien lymphoider Zellen erkennbar (Tabelle 1). Die Zellen werden bis zu 6 Monate in Kultur vermehrt, ohne daß eine Alterung der Zellen zu beobachten ist.

In Kultur C (ohne DNA-Übertragung) tritt keine Vermehrung der Lymphozyten ein und es werden keine kontinuierlich wachsenden Kolonien lymphoider Zellen erhalten. Die Zellen altern innerhalb der ersten Woche und sterben ab.

**Tabelle 1**

| Immortalisierung menschlicher Lymphozyten durch Transfektion mit klonierter pLC108 und pEFC38 DNA | | | | | |
|---|---|---|---|---|---|
| Kultur | eingesetzte Lymphozyten | transfizierte | | immortal. lymphoide Kolonien | Immort.effizienz* Kolonien/ng DNA |
| | | DNA | (ng) | | |
| A | 10⁸ | pLC108 | 200 ng | 40 | 0.2 |
| B | 10⁸ | pEFC38 | 200 ng | 50 | 0.25 |
| C | 10⁸ | - | - | 0 | 0 |

| | | | | | |
|---|---|---|---|---|---|
| * Bei der Bestimmung der Immortalisierungseffizienz wurde die unterschiedliche molare Menge der immortalisierenden DNA nicht berücksichtigt | | | | | |

### Beispiel 3

Immortalisierung menschlicher Lymphozyten durch Übertragung von enzymatisch vermehrten LC108 und EFC38 DNA Sequenzen.

Die menschlichen Lymphozyten werden wie in Beispiel 2.1 beschrieben, aus dem peripheren Blut isoliert.

Die immortalisierenden DNA-Sequenzen LC108 und EFC38 werden mit Hilfe der Polymerase-Ketten-Reaktion ("DNA polymerase chain reaction", PCR) in bekannter Weise vermehrt (Mullis und Fallona, Meth. Enzymol. 155 (1987), 335 - 350; Saiki et al., Science 239 (1988), 487 - 491).

Die Plasmid-DNA pLC108 bzw. pEFC38 wird mit Hilfe der Restriktionsendonuclease Hind III linearisiert, mit NaAcetat und Ethanol gefällt und in TE (10 mmol/l Tris, pH 7.5, 1 mmol/l EDTA) aufgenommen.

Der PCR-Reaktionsansatz (100 µl) enthält:
10 µl 10x Taq-Puffer (200 mmol/l Tris-HCl, pH 8,4, 250 mmol/l KCl, 0,5% Tween 20, 1mg/ml BSA),
1,5 µl 100 mmol/l MgCl₂,
2,5 µl dNTP-Lösung (2 mmol/l dATP, 2 mmol/l dCTP, 2 mmol/l dGTP, 2 mmol/l dTTP),
2 U Taq-Polymerase (Boehringer Mannheim),
10 ng pLC108 bzw.pEFC38 DNA, sowie
je 100 ng Primer-Oligonukleotide A und B für die Amplifikation der LC108-DNA bzw. die Primer-Oligonukleotide C und D für die Amplifikation der EFC38-DNA.

Die Primer-Oligonukleotid-Sequenzen (Primer) sind homolog zu der erfindungsgemäßen Sequenz LC108 (SEQ ID NO 1) bzw. EFC38 (SEQ ID NO 2).
Primer A: 5'GATCTTGAGTTTCCTCGTTGTAGGT 3' (SEQ ID NO 3)
Primer B: 5'GATCCAAAGCCCTCTGCTGGCCTCC 3' (SEQ ID NO 4)
Primer C: 5'GATCCAATCAGCTCAGCCACCCCCA 3' (SEQ ID NO 5)
Primer D: 5'GATCAAAACCAGGGCCTCCCACATG 3' (SEQ ID NO 6)
Es werden 30 Zyklen mit folgendem Temperaturprofil durchgeführt:
1. 1 x [5 min 95°C]
2. 30 x [30 sec 55°C; 90 sec 72°C; 60 sec 95°C]
3. 1 x [15 min 72°C]
Die so amplifizierten DNA-Sequenzen LC108 (203 bp) und EFC38 (376 bp) werden mit Chloroform/Isoamylalkohol (24:1, v/v) extrahiert, mit NaAcetat und Ethanol gefällt, in TE pH 7,5 resuspendiert und zur Immortalisierung menschlicher Lymphozyten eingesetzt.

Dazu werden gemäß Beipiel 2.1 präparierte Lymphozyten (3 x 10⁸ Zellen) mit pokeweed mitogen (5 µg/ml) zur Zellteilung angeregt und einen Tag später in drei Parallelkulturen (A, B, C) zu je 10⁸ Zellen aufgeteilt.

Die Zellen der Kultur A werden mit 10 ng der PCR-amplifizierten DNA-Sequenz LC108, die Zellen der Kultur B mit 10 ng der EFC38 DNA transfiziert. Die DNA-Übertragung und die Kultur der transfizierten Zellen erfolgt wie in Beispiel 2.2 beschrieben. Die Zellen der Kultur C werden genauso behandelt wie die Zellen der Kulturen A und B, jedoch ohne daß eine zu übertragende DNA im Inkubationsmedium vorhanden ist.

In den Kulturen A und B werden 3 bis 4 Wochen nach Transfektion Kolonien lymphoider Zellen beobachtet, die an Größe stetig zunehmen. In der 5. Woche zerfallen diese Kolonien, und es wachsen mehrere kleinere Kolonien lymphoider Zellen, die in der 6. Woche in größere Kulturgefäße übertragen werden können (Tabelle 2). Die Zellen werden routinemäßig mehrere Monate in Kultur vermehrt, ohne daß eine Zell-Alterung zu beobachten ist. In Kultur C werden keine kontinuierlich wachsenden Kolonien lymphoider Zellen erhalten. Die Zellen sterben innerhalb von 5 Tagen nach Schein-DNA-Übertragung ab.

**Tabelle 2**

| Immortalisierung menschlicher Lymphozyten durch Übertragung von enzymatisch vermehrter LC108- bzw. EFC38-DNA | | | | | |
|---|---|---|---|---|---|
| Kultur | eingesetzte Lymphozyten | transfizierte | | immortal. lymphoide Kolonien | Immortal.effizienz* Kolonien/ng DNA |
| | | DNA | (ng) | | |
| A | 10⁸ | LC108 | 10 ng | 35 | 3.5 |
| B | 10⁸ | EFC38 | 24 ng | 34 | 1.4 |
| C | 10⁸ | - | - | 0 | 0 |

| | | | | | |
|---|---|---|---|---|---|
| * Bei der Bestimmung der Immortalisierungseffizienz wurde die unterschiedliche molare Menge der immortalisierenden DNA nicht berücksichtigt | | | | | |

### Beipiel 4

Immortalisierung menschlicher Lymphozyten durch Transfektion mit einer DNA, welche zuvor mit einer Proteinfraktion aus permanent züchtbaren humanen oder tierischen Zellen inkubiert wurde.

Die immortalisierenden DNA-Sequenzen LC108 und EFC38 werden wie in Beispiel 3 beschrieben enzymatisch vermehrt. Weiterhin werden folgende Proteinfraktionen (N) und (Z) gewonnen:
Ehrlich-Aszites Zellen (10⁸ Zellen) werden in 50 mmol/l Tris, pH 7,4, 10 mmol/l EDTA, 3 mmol/l MgCl₂, resuspendiert und durch wiederholtes Einfrieren und Auftauen lysiert. Die Kerne werden von der Zytoplasma-Fraktion (Z) durch 30 minütige Zentrifugation bei 6000 x g abgetrennt. Die Zellkerne werden zur Herstellung der Kernfraktion (N) in 20 mmol/l Hepes, pH 7.9, 420 mmol/l NaCl, 1,5 mmol/l MgCl₂, 2 mmol/l EDTA, 10 mmol/l β-Mercaptoethanol, 2 µg/ ml Aprotinin, 25% (v/v) Glycerin resuspendiert. Die Kerne werden homogenisiert, 2 x 1 min mit Ultraschall behandelt und 30 min bei 25 000 x g, 4°C zentrifugiert. Der Überstand (Kern-Fraktion N) wird gegen PBS dialysiert. Die Zytoplasma-Fraktion (Z) wird 1 Std bei 100000 x g, 4°C, zentrifugiert und der Überstand gegen PBS dialysiert. Die RNA der Kern-Fraktion (N) und der Zytoplasma-Fraktion (Z) wird durch Inkubation mit RNase A (20 µg/ml) und RNase T1 (1000 U/ml) bei 37°C , 1 Std. abgebaut.

Zur Herstellung der EFC38 DNA/Protein-Partikel werden 10 ng EFC38 DNA mit jeweils 5 µl Überstand der Zytoplasma-Fraktion oder Kern-Fraktion 30 min bei 4°C in Gegenwart von NaCl (80 mmol/l), Tris-HCl (10 mmol/l), pH 7.0, β-Mercaptoethanol (10 mmol/l), tRNA (600 ng/µl), Hering-Sperma DNA (66 ng/µl), 1 mmol/l ATP, 1 mmol/l GTP inkubiert. Zur Herstellung der LC108 DNA/Protein-Partikel werden 10ng LC108 DNA in Gegenwart von ZnCl₂ (8 mmol/l), Tris-HCl (10 mmol/l), pH 7.0, β-Mercaptoethanol (10 mmol/l), tRNA (600 µg/ml), Hering-Sperma DNA (66 µg/ml) (1 mM ATP, 1 mM GTP) mit jeweils 5 µl Zytoplasma- oder Kernfraktion 30 min bei 4°C inkubiert. Die Proteine, die nicht an die jeweilige DNA-Sequenz gebunden haben, werden durch Inkubation mit Proteinase K (20 µg/ml) für 1 Stunde bei 60°C abgebaut.

Die zu immortalisierenden Lymphozyten aus dem peripheren Blut wurden wie in Beispiel 2.1 beschrieben isoliert. Die Lymphozyten werden in 9 Parallel-Kulturen zu je 10⁸ Zellen aufgeteilt. Die Zellen werden wie in Beispiel 2.2 beschrieben transfiziert: die Zellen der Kultur A mit 10 ng LC108 DNA, die Kulturen B bzw. C mit jeweils 10 ng LC108 DNA, die zuvor mit Proteinen der Zytoplasma-Fraktion (Z) bzw. Kern-Fraktion (N) inkubiert wurden; die Kultur D mit 10 ng EFC38 DNA und die Kulturen E und F mit jeweils 10 ng EFC38 DNA , die zuvor mit Proteinen der Zytoplasma-Fraktion (Z) bzw. Kern-Fraktion (N) inkubiert wurden. Die Zellen der Kultur G werden genauso behandelt wie die anderen Kulturen, jedoch ohne daß Proteine oder DNA vorhanden sind. Die Zellen der Kultur H werden mit 5 µl Zytoplasma-Fraktion (Z), die Zellen der Kultur I mit 5 µl Kern-Fraktion (N) inkubiert. Die Kultur der transfizierten und immortalisierten Zellen erfolgt wie in Beispiel 2.2 beschrieben.

In den Kulturen A bis F werden kontinuierlich wachsende Kolonien lymphoider Zellen beobachtet, die ab der 6. Woche kontinuierlich passagiert werden können, ohne daß eine Alterung der Zellen zu beobachten ist. In den Kulturen, die mit DNA/Protein-Partikeln transfiziert wurden, erhält man mehr immortalisierte Kolonien als nach Transfektion mit der jeweiligen DNA ohne Protein-Inkubation (Tabelle 3). In den Kulturen G, H und I wird keine Zellproliferation beobachtet. Die Zellen sterben innerhalb der ersten Woche nach Transfektion ab.

**Tabelle 3**

| Immortalisierung menschlicher Lymphozyten durch Übertragung von LC108 DNA/Protein- und EFC38 DNA/Protein-Partikeln | | | | | | |
|---|---|---|---|---|---|---|
| Kultur | einges. Lympho. | transfizierte | | Proteinfraktion | immortal. lymphoide Kolonien | Immortal.effizienz* Kol./ng DNA |
| | | DNA | (ng) | | | |
| A | 10⁸ | LC108 | 10 ng | - | 35 | 3.5 |
| B | 10⁸ | LC108 | 10 ng | Z | ca. 200 | 20 |
| C | 10⁸ | LC108 | 10 ng | N | ca. 170 | 17 |
| D | 10⁸ | EFC38 | 24 ng | - | 34 | 1.4 |
| E | 10⁸ | EFC38 | 24 ng | Z | ca. 90 | 3.7 |
| F | 10⁸ | EFC38 | 24 ng | N | ca. 100 | 4.2 |
| G | 10⁸ | - | - | - | 0 | 0 |
| H | 10⁸ | - | - | Z | 0 | 0 |
| I | 10⁸ | - | - | N | 0 | 0 |

| | | | | | | |
|---|---|---|---|---|---|---|
| * Bei der Bestimmung der Immortalisierungseffizienz wurde die unterschiedliche molare Menge der immortalisierenden DNA nicht berücksichtigt | | | | | | |

### Beispiel 5

Verwendung immortalisierter menschlicher Lymphozyten zur Prüfung der neoplastisch transformierenden Aktivität von alkylierenden Nitroso-Karzinogenen
Die gemäß Beispiel 2 erhaltenen immortalisierten menschlichen Lymphozyten prägen keinen neoplastisch transformierten Phänotyp aus. So bilden diese Zellen keine Kolonien in halbfestem Agar-Medium. Tumorzellen wachsen dagegen in einem solchen Medium.

Durch Inkubation der immortalisierten Zellen mit karzinogenen Substanzen in vitro kann ein Wachstum in halbfestem Agar-Medium induziert werden. Die Koloniebildung der immortalisierten Zellen in diesem Medium ist somit ein Hinweis auf eine mögliche transformierende Aktivität der entsprechenden Substanz.

### 1. Inkubation der Zellen mit der zu testenden Substanz

Immortalisierte menschliche Lymphozyten werden wie in Beispiel 2 beschrieben durch Transfektion mit LC108-DNA (SEQ ID NO 1) bzw. EFC38-DNA (SEQ ID NO 2) erhalten. Die immortalisierten Lymphozyten werden in Iscove's DMEM mit 10% fötalem Kälberserum bei 37°C und 5% CO₂ kultiviert, mit PBS gewaschen und in einer Zelldichte von 10⁶ Zellen/ml in serumfreiem Iscove's DMEM, pH 6,0 resuspendiert. Anschließend werden die Zellen mit 0,1 - 100 µg/ml) 1-Nitroso-1-harnstoff (NMU) bzw. 0,001 - 2 µg/ml N'-methyl-N-nitro-N-nitroso-guanidin (MNNG) für 30 Minuten bei 37°C inkubiert. Die Zellen werden anschließend mit PBS gewaschen.

### 2. Bestimmung der Koloniebildung in halb-festem Agar-Medium

Zur Testung der Koloniebildung in halb-festem Agar-Medium werden 3ml einer 0,5%igen Seaplaque-Agarlösung (FMC, Rockland, USA) in Iscove's DMEM mit 10% fötalem Kälberserum in eine 60mm-Petrischale gegeben. Nach Erstarren des Agars werden die gemäß 1. erhaltenen Zellen in 3ml einer 0,35%igen Agarlösung in Iscove's DMEM resuspendiert (3 Paralellansätze mit je 5 x 10⁵, 1 x 10⁶ bzw. 5 x 10⁶ Zellen in 3ml) und auf die Agarplatten gegeben. Die Zellen werden 2 bis 3 Wochen bei 37°C, 5% CO₂ inkubiert. Die in dieser Zeit angewachsenen Kolonien werden dann unter einem Stereomikroskop ausgezählt. Die Ergebnisse sind in der Tabelle 4 zusammengefaßt.

**Tabelle 4**

| Koloniebildung von immortalisierten Lymphozyten in halbfestem Agar-Medium nach Inkubation mit NMU bzw. MNNG | | | |
|---|---|---|---|
| Karzinogen | Konzentration (µg/ml) | Zahl der Kolonien im Weichagar pro eingesetzte Zellen | Klonierungseffizienz |
| --- | --- | 0 / 10⁷ Zellen | < 10⁻⁷ |
| NMU | 0.1 | 15 / 10⁶ Zellen | 1.5 x 10⁻⁵ |
| | 1.0 | 82 / 10⁶ Zellen | 8.2 x 10⁻⁵ |
| | 10 | 250 / 10⁶ Zellen | 2.5 x 10⁻⁴ |
| | 20 | 300 / 10⁶ Zellen | 3.0 x 10⁻⁴ |
| MNNG | 0.001 | 10 / 10⁶ Zellen | 1.0 x 10⁻⁵ |
| | 0.01 | 90 / 10⁶ Zellen | 9.0 x 10⁻⁵ |
| | 0.1 | 540 / 10⁶ Zellen | 5.4 x 10⁻⁴ |
| | 1.0 | 1800 / 10⁶ Zellen | 1.8 x 10⁻³ |
| | 5.0 | 4000 / 10⁶ Zellen | 4.0 x 10⁻³ |

## Patentansprüche

1. DNA, welche zur Immortalisierung von normalen humanen oder tierischen Zellen geeignet ist, dadurch erhältlich, daß man eine Mitochondrien-freie Zytoplasmafraktion aus permanent züchtbaren humanen oder tierischen Zellen gewinnt, diese Zytoplasmafraktion mit RNase und Proteinase behandelt, daraus eine Fraktion isoliert, welche in einem CsCl-Gradienten eine Dichte von etwa 1,86 g/cm³ aufweist und aus dieser DNA-Fraktion eine DNA isoliert, welche mit der in SEQ ID NO 1 und/oder SEQ ID NO 2 gezeigten DNA-Sequenz hybridisiert.

2. DNA, welche zur Immortalisierung von humanen oder tierischen Zellen geeignet ist und die in SEQ ID NO 1 oder SEQ ID NO 2 angegebene Sequenz aufweist.

3. Verfahren zur Gewinnung einer DNA, welche zur Immortalisierung von normalen humanen oder tierischen Zellen geeignet ist, dadurch gekennzeichnet, daß man eine Mitochondrienfreie Zytoplasmafraktion aus permanent züchtbaren humanen oder tierischen Zellen gewinnt, diese Zytoplasmafraktion mit RNase und Proteinase behandelt, daraus eine Fraktion isoliert, welche in einem CsCl-Gradienten eine Dichte von etwa 1,86 g/cm³ aufweist und aus dieser DNA-Fraktion eine DNA isoliert, welche mit der in SEQ ID NO 1 oder SEQ ID NO 2 gezeigten DNA-Sequenz hybridisiert.

4. Verfahren zur Gewinnung einer DNA, welche zur Immortalisierung von normalen humanen oder tierischen Zellen geeignet ist, dadurch gekennzeichnet, daß man eine genomische Gen-Bank oder eine cDNA-Bank von menschlichen oder tierischen Zellen mit der in SEQ ID NO 1 oder SEQ ID NO 2 gezeigten DNA-Sequenz hybridisiert und die mit einer dieser DNA-Sequenzen hybridisierende DNA isoliert.

5. Verfahren zur Gewinnung einer DNA, welche zur Immortalisierung von normalen humanen oder tierischen Zellen geeignet ist, dadurch gekennzeichnet, daß man eine DNA- oder RNA-Fraktion von menschlichen oder tierischen Zellen enzymatisch vermehrt, wobei Oligonukleotid-Starter Moleküle verwendet werden, welche zu der in SEQ ID NO 1 und/oder SEQ ID NO 2 gezeigten Sequenz homolog sind.

6. Verfahren nach Anspruch 5, dadurch gekennzeichnet, daß man Oligonukleotid-Starter Moleküle verwendet, welche die in SEQ ID NO 3 und SEQ ID NO 4 oder die in SEQ ID NO 5 und SEQ ID NO 6 gezeigte Sequenz aufweisen.

7. Verfahren zur Gewinnung einer DNA, welche zur Immortalisierung von normalen humanen oder tierischen Zellen geeignet ist, dadurch gekennzeichnet, daß man die in SEQ ID NO 1 oder SEQ ID NO 2 gezeigte DNA-Sequenz chemisch synthetisiert.

8. Verfahren zur Herstellung von immortalisierten humanen oder tierischen Zellen durch Transfektion von normalen humanen oder tierischen Zellen mit einer DNA-Sequenz gemäß einem der Ansprüche 1-3 oder einem Teil dieser Sequenz und Züchtung der transfizierten Zellen in einem Kulturmedium ohne Selektionssubstanzen.

9. Verfahren zur Herstellung von immortalisierten humanen oder tierischen Zellen durch Transfektion von normalen humanen oder tierischen Zellen mit einer DNA-Sequenz gemäß einem der Ansprüche 1-3, welche zuvor mit einer Proteinfraktion aus permanent züchtbaren humanen oder tierischen Zellen inkubiert wurde, wobei als Proteinfraktion ein 25000 x g Überstand der Kernfraktion oder ein 100000 x g Überstand der Zytoplasmafraktion verwendet wird, der mit RNase und Proteinase vorbehandelt wurde.

10. Verfahren zur Herstellung von immortalisierten humanen oder tierischen Zellen durch Transfektion von normalen humanen oder tierischen Zellen mit einer DNA gemäß einem der Ansprüche 1 - 3 oder einem Teil dieser Sequenz, wobei man die genannte immortalisierende DNA zunächst kloniert und dann in die zu immortalisierenden Zellen überträgt.

11. Verfahren zur Herstellung von immortalisierten humanen oder tierischen Zellen durch Transfektion von normalen humanen oder tierischen Zellen mit einer DNA gemäß einem der Ansprüche 1 - 3, wobei einzelne Basen der genannten DNA-Sequenzen durch andere oder durch modifizierte Basen ersetzt sind.

12. Verfahren zur Herstellung von immortalisierten humanen oder tierischen Zellen durch Transfektion von normalen humanen oder tierischen Zellen mit der Gesamt-DNA aus einer nach einem Verfahren gemäß einem der Ansprüche 8 - 11 erhältlichen immortalisierten Zelle.

13. Verfahren nach einem der Ansprüche 8 - 12, wobei man die zu immortalisierende Zelle in einem zur Teilung angeregten Zustand mit der DNA transfiziert.

14. Verwendung einer nach einem der Ansprüche 8 - 13 erhaltenen immortalisierten züchtbaren Zelle zur Gewinnung von homologen und heterologen Zellprodukten, zur Prüfung von Wirksubstanzen oder von Teilchen-Strahlen oder elektromagnetischen Strahlen.


SEQUENZPROTOKOLL

(1) ALLGEMEINE INFORMATION:

(i) ANMELDER:
(A) NAME: Boehringer Mannheim GmbH
(B) STRASSE: Sandhofer Str. 116
(C) ORT: Mannheim
(E) LAND: Deutschland
(F) POSTLEITZAHL: D-6800

(ii) ANMELDETITEL: DNA zur Immortalisierung von humanen oder
tierischen Zellen

(iii) ANZAHL DER SEQUENZEN: 6

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(vi) FRÜHERE ANMELDEDATEN:
(A) ANMELDENUMMER: DE P 42 18 945.4
(B) ANMELDEDATUM: 10-JUN-1992

(2) INFORMATION ZU SEQ ID NO: 1:

(i) SEQUENZ CHARAKTERISTIKA:
(A) LÄNGE: 203 Basenpaare
(B) ART: Nukleinsäure
(C) STRANGFORM: Doppel
(D) TOPOLOGIE: linear

(xi) SEQUENZBESCHREIBUNG: SEQ ID NO: 1:

GATCTTGAGT TTCCTCGTTG TAGGTCTTCC CTGGCTCTGC TCACGCTCTC ACTGACTTCT 60

CTCAGCTCAG TCACAGTGTC TATTTCTTTC CACTTAAAGA TGTGCATTTT TATTTGATGC 120

GTGCAGGTGT TTTGCCTGCA TGGATGGCTG TGCACCATGT ATGGGACCTG GTGCTCTTGG 180

AGGCCAGCAG AGGGCTTTGG ATC 203

(2) INFORMATION ZU SEQ ID NO: 2:

(i) SEQUENZ CHARAKTERISTIKA:
(A) LÄNGE: 376 Basenpaare
(B) ART: Nukleinsäure
(C) STRANGFORM: Doppel
(D) TOPOLOGIE: linear

(xi) SEQUENZBESCHREIBUNG: SEQ ID NO: 2:

GATCCAATCA GCTCAGCCAC CCCCAGCTCT CCTGTATGTA TGGCTCCAAT GCTGTTCATC 60

CCTCAGCATA AATCAATCAT TTGGTTTAGA TTCCTCCCTT TGACTTATTG CTACTATTAG 120

TATCAGTGAC TCTTCAGCCG ATTCTTTTCA GACATTGGAA CCCCAGCCTC AGATCACAGT 180

TGTAGAACAA ATATTAAAAG AGTAAATTAT TATATCATTG AACATTCAAA AGTGCTTTGC 240

AGTCATTGAC ACATAATAAT AATGAAGCCT AAACAGTAAC ATGAAAATGT GGAATTGTAT 300

TAATGTAAAA TCAAGGCCTG GGGCATAGCT CATTGGTGGA TGTTTGCCTA TCATGTGGGA 360

GGCCCTGGTT TTGATC 376

(2) INFORMATION ZU SEQ ID NO: 3:

(i) SEQUENZ CHARAKTERISTIKA:
(A) LÄNGE: 25 Basenpaare
(B) ART: Nukleinsäure
(C) STRANGFORM: Einzel
(D) TOPOLOGIE: linear

(xi) SEQUENZBESCHREIBUNG: SEQ ID NO: 3:

GATCTTGAGT TTCCTCGTTG TAGGT 25

(2) INFORMATION ZU SEQ ID NO: 4:

(i) SEQUENZ CHARAKTERISTIKA:
(A) LÄNGE: 25 Basenpaare
(B) ART: Nukleinsäure
(C) STRANGFORM: Einzel
(D) TOPOLOGIE: linear

(xi) SEQUENZBESCHREIBUNG: SEQ ID NO: 4:

GATCCAAAGC CCTCTGCTGG CCTCC 25

(2) INFORMATION ZU SEQ ID NO: 5:

(i) SEQUENZ CHARAKTERISTIKA:
(A) LÄNGE: 25 Basenpaare
(B) ART: Nukleinsäure
(C) STRANGFORM: Einzel
(D) TOPOLOGIE: linear

(xi) SEQUENZBESCHREIBUNG: SEQ ID NO: 5:

GATCCAATCA GCTCAGCCAC CCCCA 25

(2) INFORMATION ZU SEQ ID NO: 6:

(i) SEQUENZ CHARAKTERISTIKA:
(A) LÄNGE: 25 Basenpaare
(B) ART: Nukleinsäure
(C) STRANGFORM: Einzel
(D) TOPOLOGIE: linear

(xi) SEQUENZBESCHREIBUNG: SEQ ID NO: 6:

GATCAAAACC AGGGCCTCCC ACATG 25